(19) Europäisches Patentamt
European Patent Office
Office européen des brevets

(11) **EP 4 417 304 A1**

(12) **EUROPEAN PATENT APPLICATION**
published in accordance with Art. 153(4) EPC

(43) Date of publication:
**21.08.2024 Bulletin 2024/34**

(21) Application number: **22881044.6**

(22) Date of filing: **12.10.2022**

(51) International Patent Classification (IPC):
**B01J 23/888** (2006.01)     **B01J 35/10** (2006.01)
**B01J 37/08** (2006.01)     **C07B 61/00** (2006.01)
**C07C 45/35** (2006.01)     **C07C 47/22** (2006.01)
**C07C 51/235** (2006.01)     **C07C 57/055** (2006.01)

(52) Cooperative Patent Classification (CPC):
**B01J 23/888; B01J 35/60; B01J 37/08;
C07B 61/00; C07C 45/35; C07C 47/22;
C07C 51/235; C07C 57/04**

(86) International application number:
**PCT/JP2022/038037**

(87) International publication number:
**WO 2023/063349 (20.04.2023 Gazette 2023/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.10.2021 JP 2021168460**

(71) Applicant: **Nippon Kayaku Kabushiki Kaisha
Tokyo 100-0005 (JP)**

(72) Inventors:
• **YOSHIDA, Hideo**
  **Sanyoonoda-shi**
  **Yamaguchi 757-8686 (JP)**
• **KOHARA, Kento**
  **Sanyoonoda-shi**
  **Yamaguchi 757-8686 (JP)**

(74) Representative: **Gille Hrabal
Partnerschaftsgesellschaft mbB
Patentanwälte
Brucknerstraße 20
40593 Düsseldorf (DE)**

(54) **CATALYST FOR PRODUCTION OF UNSATURATED CARBOXYLIC ACID, METHOD FOR PRODUCING SAME, AND METHOD FOR PRODUCING UNSATURATED CARBOXYLIC ACID**

(57)     The present invention relates to catalysts for producing an unsaturated carboxylic acid, containing: a catalytically active component represented by the following formula (1), in which the catalysts have a median diameter D50S (mm) 5.0 mm or more and 8.0 mm or less, and a relationship between a specific surface area $A$ (m²/g) and the D50S (mm) is expressed by the following expression (2).

$$(Mo)_{12}(V)_a(W)_b(Cu)_c(Sb)_d(X)_e(Y)_f(Z)_g(O)_h \quad (1)$$

$$8.0 \leq D50S \times A \leq 20.0 \quad (2)$$

**EP 4 417 304 A1**

**Description**

TECHNICAL FIELD

The present invention relates to catalysts for producing an unsaturated carboxylic acid in a high yield by subjecting an unsaturated aldehyde to catalytic gas-phase oxidation in the presence of molecular oxygen or a molecular oxygen-containing gas, a method for producing the catalysts, and a method for producing an unsaturated carboxylic acid using the catalysts.

BACKGROUND ART

Acrylic acid is becoming increasingly important as a raw material for a water absorbent resin, an adhesive, and the like. Thus, in recent years, improved performance of a catalyst for producing acrylic acid by subjecting acrolein as a raw material to a catalytic gas-phase oxidation reaction has been demanded. Accordingly, various companies have made various improvements to catalysts that can be used to produce acrylic acid in a high yield and in a stable manner over a long period of time, and for example, the following proposals have been made.

Patent Literatures 1 to 3 disclose improvements in catalyst composition or the like focusing on X-ray diffraction peaks of a catalytically active component. These catalysts have been proposed as a catalyst that achieves high activity and a high yield.

In addition, there is always a problem of low mechanical strength of the catalyst, and various solutions have been proposed.

In Patent Literature 4, a catalyst preparation method has been devised to achieve improved activity and mechanical strength.

Patent Literature 5 discloses a technique that uses at least two types of inorganic fibers having different average diameters to improve the mechanical strength of the catalyst and to prevent powdering during filling of the catalyst.

As described above, regarding a catalyst for producing acrylic acid using acrolein as a raw material, in addition to inherently severe problems such as activity and yield, improvements in mechanical strength and prevention of powdering have also been studied. This is to prevent serious problems such as an increased pressure loss in a reactor or blockage of a reaction tube. This problem has not been sufficiently solved by the solutions disclosed in Patent Literatures 1 to 5 and the like.

CITATION LIST

PATENT LITERATURE

Patent Literature 1: JPH08-299797A
Patent Literature 2: JP2003-251184A
Patent Literature 3: JP2015-120133A
Patent Literature 4: JP2001-79408A
Patent Literature 5: WO 2012/073584

SUMMARY OF INVENTION

TECHNICAL PROBLEM

In view of the above situation, an object of the present invention is to propose catalysts that achieve suppressing a pressure loss during a catalytic gas-phase oxidation reaction using acrolein as a raw material, that is, reduction of a difference between an inlet pressure and an outlet pressure of a reactor (hereinafter, may be simply expressed as a differential pressure), and that has high activity.

SOLUTION TO PROBLEM

The inventors of the present application have made a thorough study on the above current situation and problems, and as a result, it has been found that catalysts having a specific specific surface area and specific particle size distribution parameters can contribute to reducing a differential pressure during a catalytic gas-phase oxidation reaction and can maintain high activity.

**EP 4 417 304 A1**

That is, the present invention relates to the following 1) to 12).

1) Catalysts for producing an unsaturated carboxylic acid, containing:

a catalytically active component represented by the following formula (1), in which
the catalysts have a median diameter D50S (mm) 5.0 mm or more and 8.0 mm or less, and
a relationship between a specific surface area A (m²/g) of the catalysts and the median diameter D50S (mm) of the catalysts is expressed by the following expression (2):

$$(Mo)_{12}(V)_a(W)_b(Cu)_c(Sb)_d(X)_e(Y)_f(Z)_g(O)_h \qquad (1)$$

$$8.0 \leq D50S \times A \leq 20.0 \qquad (2)$$

(in the formula (1), Mo, V, W, Cu, Sb, and O represent molybdenum, vanadium, tungsten, copper, antimony, and oxygen respectively; X represents at least one element selected from the group consisting of an alkali metal and thallium; Y represents at least one element selected from the group consisting of magnesium, calcium, strontium, barium, and zinc; and Z represents at least one element selected from the group consisting of niobium, cerium, tin, chromium, manganese, iron, cobalt, samarium, germanium, titanium, and arsenic, in addition, a, b, c, d, e, f, g, and h indicate an atomic ratio of respective element to a molybdenum atom being 12, and $0 < a \leq 10$, $0 \leq b \leq 10$, $0 < c \leq 6$, $0 \leq d \leq 10$, $0 \leq e \leq 0.5$, $0 \leq f \leq 1$, and $0 \leq g < 6$ are satisfied, further, h is the number of oxygen atoms required to satisfy a valence of each component described above).

2) The catalysts for producing an unsaturated carboxylic acid according to the above 1), in which the relationship between the specific surface area A (m²/g) and the median diameter D50S (mm) is expressed by the following expression (3):

$$8.6 \leq D50S \times A \leq 20.0 \qquad (3).$$

3) The catalysts for producing an unsaturated carboxylic acid according to above 1) or 2), in which a cumulative pore volume B (cm³/g) measured by a nitrogen adsorption method is $9.70 \times 10^{-4}$ cm³/g or more and $2.00 \times 10^{-3}$ cm³/g or less.
4) The catalysts for producing an unsaturated carboxylic acid according to any one of the above 1) to 3), $1 \leq a \leq 5$, $0.5 \leq b \leq 3$, $0.5 \leq c \leq 3$, and $0 < d \leq 2$ are satisfied in the formula (1).
5) The catalysts for producing an unsaturated carboxylic acid according to any one of the above 1) to 4), in which catalyst precursors containing the catalytically active component are carried on inert carriers.
6) The catalysts for producing an unsaturated carboxylic acid according to the above 5), in which the inert carrier is silica, alumina, or a combination thereof.
7) The catalysts for producing an unsaturated carboxylic acid according to any one of the above 1) to 6), further containing:
an inorganic fiber.
8) A method for producing the catalysts for producing an unsaturated carboxylic acid according to the above 5) or 6), including:

carrying the catalyst precursors on the inert carriers, in which
the catalyst precursors have a median diameter D50T of 1.0 μm or more and 14.0 μm or less.

9) The method for producing the catalysts for producing an unsaturated carboxylic acid according to the above 8), further including:
carrying out calcination at a temperature of 350°C or higher and 380°C or lower, after carrying the catalyst precursors on the inert carriers.
10) A method for producing an unsaturated carboxylic acid using the catalysts for producing an unsaturated carboxylic acid according to any one of the above 1) to 7).
11) A method for producing an unsaturated carboxylic acid including using a reaction tube filled with two or more of the catalysts for producing an unsaturated carboxylic acid according to any one of above 1) to 7) in multiple layers.
12) The method for producing an unsaturated carboxylic acid according to the above 10) or 11), in which a difference

3

between a reactor inlet pressure and a reactor outlet pressure during a reaction is 27 kPa or less.

ADVANTAGEOUS EFFECTS OF INVENTION

According to the present invention, when producing acrylic acid by subjecting acrolein as a raw material to a catalytic gas-phase oxidation reaction, the differential pressure can be reduced and the catalytic activity can be maintained at a high level.

DESCRIPTION OF EMBODIMENTS

[Median Diameter D50S (mm)]

Catalysts according to the present invention have a median diameter D50S of 5.0 mm or more and 8.0 mm or less.
The median diameter is a particle diameter that is a median value of outer diameters of the catalyst. The median diameter is determined by measuring outer diameters of 200 catalyst particles using, for example, a caliper (trade name "ABS Digimatic Caliper CD-AX", manufactured by Mitutoyo Corporation), and taking the median value thereof. That is, D50S means the median diameter D50 derived from a particle size distribution based on the number of the particles. The shape of the catalyst is not limited, and thus, for example, for non-spherical particles such as a ring shape, a column shape, or a tablet shape, the longest part of the particle is measured. The median diameter is typically expressed as D50, but in the present description, it is expressed as D50S for distinguishing it from a median diameter of catalyst precursors to be described below.
A lower limit of a preferred range of the median diameter D50S is 5.1 mm, is 5.2 mm, 5.3 mm, 5.4 mm, 5.5 mm, 5.6 mm, 5.7 mm, 5.8 mm, 5.9 mm, and 6.0 mm in order of preference, and is particularly preferably 6.4 mm. In addition, a preferred upper limit is 7.9 mm, is 7.6 mm, 7.4 mm, 7.2 mm, 7.0 mm, and 6.8 mm in order of preference, and is particularly preferably 6.6 mm. That is, the median diameter of the catalysts is most preferably 6.4 mm or more and 6.6 mm or less.
A reduction in differential pressure during a catalytic gas-phase oxidation reaction, which is one of the objects of the present invention, can be achieved by reducing an amount of the catalysts for filling a reaction tube, that is, by increasing the median diameter D50S. However, when the median diameter D50S is increased, reaction opportunities are reduced due to a reduction in the amount of the catalyst for filling, and a raw material conversion rate is remarkably reduced. Therefore, the present inventors have studied various conditions and have found that when a specific relationship is present between a specific surface area A described below and the median diameter D50S, a reduction in differential pressure and catalytic activity can be achieved at a high level in a well-balanced manner.
Examples of a method of adjusting the median diameter D50S include a method of adjusting a particle diameter of inert carriers during molding, and a method of adjusting an amount of catalyst precursors to be carried on the inert carriers. The method of adjusting a particle diameter of inert carriers is particularly effective.

[Relationship between Specific Surface Area A ($m^2$/g) and D50S]

That is, in the present invention, the relationship between the specific surface area A and the median diameter D50S of the catalysts satisfies the above expression (2).
When the expression (2) is satisfied, both a reduction in differential pressure during a catalytic gas-phase oxidation reaction and a high catalytic activity can be achieved at a higher level.
Here, the specific surface area A represents the specific surface area of the catalysts measured by a BET method using a nitrogen gas adsorption method. The specific surface area A can be measured using various commercially available measuring devices, and can be measured, for example, by the following method. That is, a sample having a volume of 0.05 mL to 3.0 mL is charged into a sample tube having an inner diameter of 14 mm and pretreated under conditions of 300°C for 2 hours or longer, then measurement is carried out using a gas adsorption amount measuring device (Belsorp-Max2 (manufactured by MicrotracBEL Corp.)) under the conditions of adsorption gas species of nitrogen, a nitrogen molecule diameter of 0.364 nm, a relative pressure ratio of 0.02 to 0.4, an adsorption temperature of -196°C, and a pore diameter range for measurement of 0.7 nm to 400 nm. The measurement results are analyzed by the BET method to obtain the specific surface area A ($m^2$/g).
The preferred value of a lower limit of a product (D50S $\times$ A) of the specific surface area A and the D50S is 8.50, 8.60, 8.80, 9.00, 9.30, 9.50, 10.0, 10.5, 11.0, and 12.0 in order, and is particularly preferably 13.0. In addition, the preferred value of an upper limit is 19.0, 18.5, 18.0, and 17.5 in order, and is particularly preferably 17.0. Therefore, the value of D50S $\times$ A is most preferably 13.0 or more and 17.0 or less.
The preferred lower limit of the specific surface area A is 1.60, 1.62, 1.63, 1.64, 1.65, 1.66, 1.70, 1.72, 1.74, 1.75, 1.80, 1.90, 2.00, 2.10, 2.20, 2.30, and 2.40 in order, and is particularly preferably 2.50. The upper limit of the specific surface area A is 4.00, 3.50, 3.00, 2.90, 2.80, and 2.70 in order of preference, and particularly preferably 2.60. Therefore, the most preferred range of the specific surface area A is 2.50 or more and 2.60 or less.

Examples of a method of adjusting the above specific surface area A include a method of molding using finely powdered catalyst precursors or a method of adding a pore-forming agent during molding, and the method of molding using finely powdered catalyst precursors is particularly effective.

From the viewpoint of an acrolein conversion rate, a value (D50S/A) obtained by dividing the D50S (mm) by the specific surface area A ($m^2/g$) is preferably 2.0 or more and 3.2 or less. The preferred lower limit of the D50S/A is 2.0, 2.1, 2.2, 2.3, and 2.4 in order, and particularly preferably 2.5. The upper limit of the D50S/A is 3.2, 3.0, 2.8, and 2.7 in order of preference, and particularly preferably 2.6. Therefore, the most preferred range of the D50S/A is 2.5 or more and 2.6 or less.

[Catalyst Composition]

The catalyst according to the present invention has a composition represented by the following formula (1).

$$(Mo)_{12}(V)_a(W)_b(Cu)_c(Sb)_d(X)_e(Y)_f(Z)_g(O)_h \qquad (1)$$

(In the formula, Mo, V, W, Cu, Sb, and O represent molybdenum, vanadium, tungsten, copper, antimony, and oxygen respectively; X represents at least one element selected from the group consisting of an alkali metal and thallium; Y represents at least one element selected from the group consisting of magnesium, calcium, strontium, barium, and zinc; and Z represents at least one element selected from the group consisting of niobium, cerium, tin, chromium, manganese, iron, cobalt, samarium, germanium, titanium, and arsenic. In addition, a, b, c, d, e, f, g, and h indicate an atomic ratio of respective element to a molybdenum atom being 12, and $0 < a \leq 10$, $0 \leq b \leq 10$, $0 < c \leq 6$, $0 \leq d \leq 10$, $0 \leq e \leq 0.5$, $0 \leq f \leq 1$, and $0 \leq g < 6$ are satisfied. Further, h is the number of oxygen atoms required to satisfy a valence of each component described above.)

From the viewpoint of a selectivity and a yield, preferred ranges of a to g in the above formula (1) are as follows.

A lower limit of a is 0.2, 0.5, 0.8, 1.0, 1.5, 2.0, 2.2, and 2.5 in order of preference, and is most preferably 2.8. An upper limit of a is 9.0, 8.0, 7.0, 6.0, 5.0, 4.5, 4.0, and 3.5 in order of preference, and is most preferably 3.2. That is, the most preferred range of a is $2.8 \leq a \leq 3.2$.

A lower limit of b is 0.1, 0.2, 0.3, 0.4, 0.5, 0.6, 0.7, 0.8, and 0.9 in order of preference, and is most preferably 1.0. An upper limit of b is 9.0, 8.0, 7.0, 6.0, 5.0, 4.0, 3.0, 2.5, 2.0, and 1.5 in order of preference, and is most preferably 1.4. That is, the most preferred range of a is $1.0 \leq b \leq 1.4$.

A lower limit of c is 0.1, 0.2, 0.3, 0.4, 0.5, 0.6, 0.7, 0.8, and 0.9 in order of preference, and is most preferably 1.0. An upper limit of c is 5.0, 4.0, 3.0, 2.5, 2.0, and 1.5 in order of preference, and is most preferably 1.4. That is, the most preferred range of a is $1.0 \leq c \leq 1.4$.

A lower limit of d is 0.11, 0.15, 0.18, 0.20, 0.25, 0.30, and 0.35 in order of preference, and is most preferably 0.40. An upper limit of d is 9.0, 8.0, 7.0, 6.0, 5.0, 4.0, 3.0, 2.5, 2.0, 1.5, and 1.0 in order of preference, and is most preferably 0.70. That is, the most preferred range of d is $0.40 \leq d \leq 0.70$.

An upper limit of e is 0, 4, 0.3, 0.2, and 0.1 in order of preference. That is, the most preferred range of e is $0 \leq e \leq 0.1$.

An upper limit of f is 0.8, 0.5, 0.2, and 0.15 in order of preference, and is most preferably 0.10. That is, the most preferred range of f is $0 \leq f \leq 0.10$.

An upper limit of g is 5, 4, 3, 2, and 1 in order of preference. That is, the most preferred range of g is $0 \leq g \leq 1$.

Particularly preferably, e, f, and g are 0.

[Cumulative Pore Volume B ($cm^3/g$)]

Preferably, the catalysts according to the present invention have a cumulative pore volume B ($cm^3/g$) measured by a nitrogen adsorption method of $9.70 \times 10^{-4}$ $cm^3/g$ or more and $2.00 \times 10^{-3}$ $cm^3/g$ or less, from the viewpoint of the acrolein conversion rate.

Herein, the cumulative pore volume B represents the maximum cumulative volume of the catalyst measured by the BET method using a nitrogen gas adsorption method, and can be easily measured using various commercially available measuring devices. For example, it can be measured by the following method. That is, one of the examples is a method for obtaining the cumulative pore volume B of the catalysts by charging a sample having a volume of 0.05 mL to 3.0 mL into a sample tube having an inner diameter of 14 mm and pretreating the sample under conditions of 300°C for 2 hours or longer, then carrying out measurement using a gas adsorption amount measuring device (Belsorp-Max2 (manufactured by MicrotracBEL Corp.)) under the conditions of adsorption gas species of nitrogen, a nitrogen molecule diameter of 0.364 nm, a relative pressure ratio of 0.02 to 0.4, an adsorption temperature of -196°C, and a measured pore diameter range of 0.7 nm to 400 nm, and analyzing the measurement results by the BET method.

The more preferred lower limit of the cumulative pore volume B is $9.80 \times 10^{-4}$ cm$^3$/g, more preferably $9.90 \times 10^{-4}$ cm$^3$/g, and particularly preferably $1.00 \times 10^{-3}$ cm$^3$/g. The more preferred upper limit is $1.80 \times 10^{-3}$ cm$^3$/g, more preferably $1.60 \times 10^{-3}$ cm$^3$/g, and particularly preferably $1.50 \times 10^{-3}$ cm$^3$/g. That is, the cumulative pore volume B of the catalyst is most preferably $1.00 \times 10^{-3}$ cm$^3$/g or more and $1.50 \times 10^{-3}$ cm$^3$/g or less.

[Median Diameter D50T ($\mu$m) of Catalyst Precursors]

In the present invention, in order to set the specific surface area A (m$^2$/g) of the above catalyst to the preferred value, a median diameter D50T ($\mu$m) of the catalyst precursors is preferably 1.0 $\mu$m or more and 14.0 $\mu$m or less. The catalyst precursors are granules before the steps of molding the catalyst, and are obtained by mixing a slurry, which is obtained by mixing raw materials containing various elements constituting the catalyst, into granules using, for example, drum drying, spray drying, and evaporation to dryness, and then carrying out preliminary calcination. The catalyst precursors are also referred to as a preliminarily calcined powder in the present invention. As a drying method, particularly preferred is spray drying in which the slurry can be dried into granules in a short period of time.

The median diameter D50T of the catalyst precursors can be adjusted, for example, by appropriately adjusting spray drying conditions, and can also be adjusted by appropriately adjusting the condition of a pulverization treatment when the obtained catalyst precursors are subjected to the pulverization treatment. In the case of performing molding after performing the pulverization treatment, the median diameter of the catalyst precursors (preliminarily calcined powder) means the median diameter after carrying out the pulverization treatment.

The lower limit of a preferred range of the median diameter D50T of the catalyst precursor is 1.50 $\mu$m, 2.00 $\mu$m, 5.00 $\mu$m, 7.00 $\mu$m, and 8.00 $\mu$m in order of preference, and is particularly preferably 9.00 $\mu$m. The upper limit is 13.50 $\mu$m, 13.00 $\mu$m, 12.50 $\mu$m, 12.00 $\mu$m, 11.50 $\mu$m, 11.00 $\mu$m, and 10.50 $\mu$m in order of preference, and is particularly preferably 10.00 $\mu$m. That is, the median diameter of the catalyst precursors is most preferably 9.00 mm or more and 10.00 mm or less.

The median diameter D50T of the catalyst precursors is the particle diameter at which the cumulative volume fraction is 50%, and is determined by measuring the particle size distribution using, for example, a laser diffraction scattering particle size distribution measuring device (trade name "LMS-2000e", manufactured by SEISHIN ENTERPRISE Co., Ltd.), and determining the volume average. That is, the median diameter D50T is a median value in terms of volume fraction. There is no problem as long as the measurement conditions are general conditions known to those skilled in the art. For example, a scattering range is 10% to 20%, a measurement time is 3 seconds, the number of snaps is 3000, a sample material is Fraunhofer, a dispersion medium is water, a stirrer pump is set to 2500 rpm, and the measurement is carried out without applying ultrasonic waves.

[Carrying]

Catalysts in which a preliminarily calcined powder, which is obtained by preliminary calcination after preparation of the catalytically active component, is carried on inert carriers are particularly effective in the effect of reducing the differential pressure.

As a material of the inert carrier, known substances such as alumina, silica, titania, zirconia, niobia, silica alumina, silicon carbide, carbides, and mixtures thereof can be used. Further, the particle diameter, water absorption, mechanical strength, crystallinity of each crystal phase, and mixing proportion are not limited, and an appropriate range can be selected in consideration of the final catalyst performance, moldability, production efficiency, and the like. The mixing proportion of the carrier to the preliminarily calcined powder is basically calculated as a active mass ratio based on the charged mass of each raw material according to the following equation. When additives such as a molding aid and a strength improver are contained, amounts thereof are included in the total amount (denominator).

Active mass ratio (mass%) = (mass of preliminarily calcined powder for use in molding)/{(mass of preliminarily calcined powder for use in molding) + (mass of carrier for use in molding)} $\times$ 100

The preferred upper limit of the above active mass ratio is 80 mass%, and more preferably 60 mass%.
The preferred lower limit is 20 mass%, and more preferably 30 mass%. That is, the most preferred range of the active mass ratio is 30 mass% or more and 60 mass% or less.
The inert carrier is preferably silica and/or alumina, and particularly preferably a mixture of silica and alumina.
A binder is preferably used for carrying. Specific examples of the binder to be used include water, ethanol, methanol, propanol, a polyhydric alcohol, polyvinyl alcohol that is a polymer-based binder, and a silica sol aqueous solution that is an inorganic binder. Ethanol, methanol, propanol, and a polyhydric alcohol are preferred, a diol such as ethylene glycol and a triol such as glycerin are preferred, and an aqueous solution having a concentration of glycerin of 5 mass% or more is preferred. Using an appropriate amount of a binder allows for providing a high-performance catalyst having good moldability and high mechanical strength. The amount of these binders to be used is typically 2 to 60 parts by mass with respect to 100 parts by mass of the preliminarily calcined powder, and 10 to 30 parts by mass is preferred in the case of using the glycerin aqueous solution. The binder and the preliminarily calcined powder may be alternately supplied to a molding machine or simultaneously supplied to the molding machine during carrying.

The carried catalysts obtained as described above can be directly supplied as catalysts for the catalytic gas-phase oxidation reaction, and the catalyst is preferably subjected to calcination as the catalytic activity may be improved. The calcination method and the calcination conditions are not limited, and known treatment methods and conditions can be applied. The optimum calcination conditions vary depending on the raw material for catalyst to be used, the catalyst composition, the preparation method, and the like, and the calcination temperature is typically 100°C to 450°C, preferably 270°C to 420°C, particularly preferably 350°C to 380°C and the calcination time is typically 1 to 20 hours. The calcination is typically carried out in an air atmosphere, and may be carried out in an atmosphere of an inert gas such as nitrogen, carbon dioxide, helium, or argon, or the calcination may be carried out in an air atmosphere after calcination in an inert gas atmosphere, if necessary.

Using the catalyst according to the present invention in a reaction that produces a corresponding unsaturated carboxylic acid using an unsaturated aldehyde such as acrolein and methacrolein as a raw material, particularly in a reaction that produces acrylic acid by subjecting acrolein to catalytic gas-phase oxidation with molecular oxygen or a molecular oxygen-containing gas, allows for achieving improvement in catalytic activity and the reduction in differential pressure, and is very effective as compared with the known method. In addition, improvement in process stability of a partial oxidation reaction accompanied by heat generation such as reduction of a hotspot temperature can also be expected. Further, the catalyst according to the present invention is also effective in reducing by-products that may adversely influence the environment and the quality of the final product, such as carbon monoxide (CO), carbon dioxide ($CO_2$), acetaldehyde, acetic acid, and formaldehyde.

In a method for producing an unsaturated carboxylic acid according to the present invention, a method for distributing a raw material gas is not limited but may be an ordinary single distribution method or a recycling method. A mixed gas containing, as starting raw materials, for example, 1 vol% to 10 vol% and preferably 4 vol% to 9 vol% of an unsaturated aldehyde, 3 vol% to 20 vol% and preferably 4 vol% to 18 vol% of molecular oxygen, 0 vol% to 60 vol% and preferably 4 vol% to 50 vol% of water vapor, and 20 vol% to 80 vol% and preferably 30 vol% to 60 vol% of an inert gas such as carbon dioxide and nitrogen at room temperature is introduced, at 250°C to 450°C under a normal pressure to 10 atm and a space velocity of 300 h$^{-1}$ to 5000 h$^{-1}$, into the catalyst according to the present invention filled in a reaction tube, and the reaction is carried out.

When the catalyst according to the present invention is used, the differential pressure can be reduced, and the reaction can be carried out at a difference between an inlet pressure and an outlet pressure of the reactor of 27 kPa or less, more preferably 25 kPa or less, and particularly preferably 22 kPa or less.

[Inorganic Fiber]

The catalyst according to the present invention preferably contains an inorganic fiber for purposes of improving mechanical strength or the like. A material of the inorganic fiber is not limited, but for example, a glass fiber, a ceramic fiber, a metal fiber, a mineral fiber, a carbon fiber, and various whiskers can be used. Among these, a glass fiber is particularly preferred.

A fiber length of the inorganic fibers is not limited as long as it does not impair the effects of the present invention, but an average fiber length is preferably about 1 μm to 1000 μm, and more preferably about 10 μm to 500 μm.

Further, two or more types of inorganic fibers can be used together. Two or more types of different materials may be used, or two or more types of the same material with different average fiber lengths may be used.

[Method for Producing Catalyst, etc.]

Specific steps for obtaining the catalyst according to the present invention are exemplified below.

Step a) preparation

Examples of raw materials for each element constituting the catalyst are as follows. When ammonium molybdate is used as a molybdenum component raw material, a high-performance catalyst can be obtained. As raw materials for tungsten, vanadium, antimony, copper and other elements, typically, an oxide, or an ammonium salt, a carbonate salt, an organic acid salt, and a hydroxide, which can be converted into an oxide by high heat, or mixtures thereof can be used. For example, a vanadium component raw material, a molybdenum component raw material, and antimony component raw material are mixed in a desired ratio with a separately prepared aqueous solution or slurry of a tungsten component raw material and a Z component raw material under conditions of 20°C to 90°C, and the mixture is heated and stirred under conditions of 20°C to 90°C for about 1 hour. Then, an aqueous solution in which a copper component raw material is dissolved and, if necessary, an X component raw material and a Y component raw material are added to obtain an aqueous solution or slurry containing catalyst components. Hereinafter, the aqueous solution or slurry containing the catalyst components will be collectively referred to as a prepared liquid (A). X represents at least one element selected from the group consisting of an alkali metal and thallium; Y represents at least one element selected from the group consisting of magnesium, calcium, strontium, barium, and zinc; and Z represents at least one element selected from the group consisting of niobium, cerium, tin, chromium, manganese, iron, cobalt, samarium, germanium, titanium, and arsenic. Here, the prepared liquid (A) is not always necessary to contain all the catalyst constituent elements, and some elements or some amounts thereof may be added in the subsequent steps. In addition, in the case of adding acids such as sulfuric acid, nitric acid, hydrochloric acid, tartaric acid, and acetic acid to dissolve each component raw material when preparing the prepared liquid (A), an insufficient acid concentration in the aqueous solution to dissolve the raw materials (for example, an appropriate concentration in a range of 5 mass% to 99 mass%) may cause a problem that the prepared liquid (A) becomes in the form of a clay-like lump, and this may fail to obtain an excellent catalyst. Therefore, the form of the obtained prepared liquid (A) is preferably an aqueous solution or slurry as an excellent catalyst can be obtained.

Step b) drying

Next, the prepared liquid (A) obtained above is dried to form a dry powder. The drying method is not limited as long as it is a method capable of completely drying the prepared liquid, and examples thereof include drum drying, freeze drying, spray drying, and evaporation to dryness. Among these, in the present invention, spray drying is particularly preferred since the slurry can be dried into a powder or granules in a short period of time. The drying temperature in the spray drying varies depending on a concentration of the slurry, a liquid feeding rate, or the like, and the temperature at the outlet of a dryer is typically 70°C to 150°C. In addition, it is preferable to dry the powder such that the average particle diameter of the obtained dry powder is 1.0 $\mu$m to 700 $\mu$m, and more preferably 20 $\mu$m to 700 $\mu$m. In this way, a dry powder (B) is obtained.

Step c) preliminary calcination

Next, the dry powder (B) obtained above is subjected to preliminary calcination. When the dry powder (B) is calcined under air circulation at 200°C to 500°C, preferably at 300°C to 400°C, the moldability, the mechanical strength, and the catalyst performance of the catalyst tend to improve. The calcination time is preferably 1 hour to 12 hours. In this way, a preliminarily calcined product (C) is obtained.

Step d) pulverization

In the above step c), the preliminarily calcined product (C) may be obtained as a solid matter (D) in which the dry powder (B) is aggregated by preliminary calcination. The solid matter (D) is pulverized to obtain a preliminarily calcined powder (E) required in the next molding step. The pulverization method is not limited, but examples thereof include a roller mill, a jet mill, a hammer mill, a rotary mill, and a vibration mill. The average particle diameter of the preliminarily calcined powder (E) obtained at this time is preferably 1.0 $\mu$m or more and 14 $\mu$m or less, and more preferably 1.0 $\mu$m or more and 10 $\mu$m or less.

In the present description of this application, the preliminarily calcined powder (E) after pulverization is described as a catalyst precursor, but when there is no aggregation in the stage of the preliminarily calcined product (C) and the preliminarily calcined product (C) can be used in the molding step without going through the pulverization step, the preliminarily calcined product (C) may be used as a catalyst precursor.

Step e) molding

Next, the preliminarily calcined powder (E) is molded. A molding method is not limited, but in the case of molding into a cylinder or ring shape, a method using a tablet molding machine, an extrusion molding machine, or the like is preferred. More preferably, in the case of molding into a spherical shape, the preliminarily calcined powder (E) may be molded into a spherical shape using a molding machine, and a method of carrying the preliminarily calcined powder (E) (containing a molding aid and a strength improver if necessary) on a carrier such as an inert ceramic is preferred. Here, as a carrying method, a tumbling granulation method, a method using a centrifugal fluid coating device, and a wash coating method are widely known, and the method is not limited as long as the preliminarily calcined powder (E) can be uniformly carried on the carrier. In consideration of the production efficiency of the catalyst and the performance of the prepared catalyst, more preferred is a method of rotating a flat or uneven disc at a high speed in a device that has the disc at the bottom of a fixed cylindrical container, to vigorously agitate a carrier charged in the container by rotation and revolution movements of the carrier, and carrying the powder component on the carrier by adding, to the container, the preliminarily calcined powder (E), and if necessary a molding aid and/or a strength improver, and a pore-forming agent. A binder is preferably used for carrying. Specific examples of the binder to be used include water, ethanol, methanol, propanol, a polyhydric alcohol, polyvinyl alcohol that is a polymer-based binder, and a silica sol aqueous solution that is an inorganic binder. Ethanol, methanol, propanol, and a polyhydric alcohol are preferred, a diol such as ethylene glycol and a triol such as glycerin are more preferred. Using an appropriate amount of a binder allows for providing a high-performance catalyst having good moldability and high mechanical strength. Specifically, a particularly high-performance catalyst can be obtained when an aqueous solution of glycerin having a concentration of 5 mass% or more is used. The amount of these binders to be used is typically 2 to 80 parts by mass with respect to 100 parts by mass of the preliminarily calcined powder (E). The inert carrier typically has a particle diameter of about 2 mm to 8 mm, and the preliminarily calcined powder (E) is carried thereon. The active mass ratio is determined in consideration of the catalyst usage conditions, for example, reaction conditions such as the space velocity of the reaction raw materials and the concentration of the raw materials, and is typically 20 mass% to 80 mass%. Here, the active mass ratio is expressed as in the following equation (4) when a molding aid, a strength improver and the like are used in molding. In this way, a molded body (F) is obtained.

In addition, as found by the present inventors, it is difficult to maintain the mechanical strength in the present invention. Therefore, an inert inorganic fiber is preferably added as a strength improver, and particularly preferably, a glass fiber is used during carrying and molding. The amount of these fibers to be used is typically 1 to 30 parts by mass with respect to 100 parts by mass of the catalytically active component solid.

Equation (4)

Active mass ratio (mass%)

$= 100 \times$ [mass of preliminarily calcined powder (E) for use in molding/(mass of preliminarily calcined powder (E) for use in molding + mass of inert carrier for use in molding + masses of molding aid and strength improver for use in molding)]

Step f) main calcination

When the molded body (F) is calcined at a temperature of 100°C to 450°C for about 1 to 12 hours, the catalytic activity and the useful yield tend to improve. The calcination temperature is preferably 270°C or higher and 420°C or lower, and more preferably 350°C or higher and 380°C or lower. Air is convenient and preferred as a gas to be circulated. In addition, nitrogen, carbon dioxide, a nitrogen oxide-containing gas for creating a reducing atmosphere, an ammonia-containing gas, a hydrogen gas, and mixtures thereof can be also used as inert gases. In this way, catalysts (G) are obtained.

EXAMPLES

Hereinafter, the present invention will be described in more detail with reference to Examples. In Examples, the raw material conversion rate, the useful yield, the useful selectivity, and the active mass ratio were calculated according to the following equations.

Raw material conversion rate (%) = (number of moles of propylene reacted)/(number of moles of propylene supplied) $\times$ 100

Useful yield (%) = (total number of moles of acroline and acrylic acid produced)/(number of moles of propylene supplied) $\times$ 100

Useful selectivity (%) = (total number of moles of acrolein and acrylic acid produced)/(number of moles of propylene

reacted) $\times$ 100

Although Examples are shown below by giving specific examples, the present invention is not limited to Examples unless it deviates from the spirit thereof.

[Example 1]

<Production of Catalysts 1>

In 2300 parts by mass of pure water heated to 95°C, 65 parts by mass of ammonium paratungstate was completely dissolved. While stirring this solution, 72.8 parts by mass of ammonium metavanadate, 440 parts by mass of ammonium molybdate, and 31 parts by mass of antimony acetate were then gradually added and the resultant solution was thoroughly stirred. Next, a solution obtained by completely dissolving 62 parts by mass of copper sulfate in 186 parts by mass of pure water heated to 60°C was added to the above solution, followed by mixing with stirring. The prepared liquid (A) was dried by a spray drying, and the obtained dry powder (B) was subjected to preliminary calcination to a temperature of 350°C after 12 hours to obtain a preliminarily calcined product (C). The obtained preliminarily calcined product (C) was pulverized with a vibration mill to obtain a preliminarily calcined powder (E). The median diameter of the obtained preliminarily calcined powder (E) was 9.44 $\mu$m. 5 mass% of crystalline cellulose and 5 mass% of a glass fiber were added to the preliminarily calcined powder (E) and mixed thoroughly, and then a tumbling granulation was performed using a 20 mass% glycerin solution as a binder to carry and mold the obtained product into a spherical shape on inert spherical carriers made of a mixture of silica and alumina such that the carrying amount was 40 mass%.
Next, main calcination was carried out such that the temperature after 12 hours was 375°C to obtain spherical catalysts 1 according to the present invention having a median diameter D50S of 6.5 mm, a specific surface area A of 2.54 m$^2$/g, D50S $\times$ A of 16.51, and D50S/A of 2.56.
The composition of the catalysts 1 is $Mo_{12}V_3W_{1.2}Cu_{1.2}Sb_{0.5}$.

<Production of Catalysts 2>

Spherical catalysts were produced in the same production method as that for the catalysts 1 except that the carrying and molding was carried out such that the median diameter D50S of the catalyst was 5.5 mm. The specific surface area A was 2.52 m$^2$/g, the D50S $\times$ A was 13.86, and the D50S/A was 2.18.

<Acrylic Acid Production Evaluation>

An acrolein oxidation reaction (second stage) was carried out after a propylene oxidation reaction (first stage), and the acrolein conversion rate was determined. In both the first stage and the second stage, a stainless steel reaction tube having an inner diameter of 25.0 mm was filled with 1.4 L of catalysts. In the first stage, a mixed gas containing 8 vol% of propylene, 70 vol% of air, 5 vol% of water vapor, and 16 vol% of nitrogen was introduced for a contact time of 2 seconds to carry out the propylene oxidation reaction to produce acrolein. In the second stage, an inlet side (upper layer) of the reaction tube was filled with the catalysts 1 diluted to 75% with alumina spheres, and a rear side of the catalysts 1 (lower layer) was filled with the catalysts 2. The gas produced in the reaction in the first stage was introduced into the second stage, the salt bath temperature (reaction temperature) in the second stage was set at 283°C, and the acrolein conversion rate was determined. A difference (differential pressure) between the inlet pressure and the outlet pressure of the reaction tube in the second stage was measured.
Table 1 shows evaluation results regarding the acrolein conversion rate (catalytic activity) and the differential pressure.

[Comparative Example 1]

<Production of Catalysts 3>

Spherical catalysts were produced in the same production method as that for the catalysts 1 except that the preliminarily calcined powder (E) was pulverized with a rotary mill such that the median diameter was 14.25 $\mu$m, and the carrying and molding was carried out such that the median diameter D50S of the catalyst was 4.9 mm. The specific surface area A was 1.74 m$^2$/g, the D50S $\times$ A was 8.53, and the D50S/A was 2.82.

<Acrylic Acid Production Evaluation>

Catalysts 4 were made by diluting the catalysts 3 to 65% with alumina spheres, the lower layer was filled with the catalysts 3 and the upper layer was filled with the catalysts 4 in the same manner as in Example 1, the acrolein oxidation reaction was carried out, and the acrolein conversion rate was determined. Table 1 shows evaluation results regarding the acrolein conversion rate (catalytic activity) and the differential pressure.

[Table 1]

| | | Upper layer | Lower layer | Evaluation | | |
|---|---|---|---|---|---|---|
| | | | | Reaction bath temperature | Acrolein conversion rate | Differential pressure |
| Example 1 | Catalyst | Catalysts 1 | Catalysts 2 | 283°C | 99.63% | 22 kPa |
| | Particle diameter D50S [mm] | 6.5 | 5.5 | | | |
| | Specific surface area A [m2/g] | 2.54 | 2.52 | | | |
| | D50S × A | 16.51 | 13.86 | | | |
| | Cumulative pore volume B [cm3/g] | 1.41E-03 | 1.40E-03 | | | |
| | Preliminarily calcined powder particle diameter D50T [μm] | 9.44 | 9.44 | | | |
| Comparative Example 1 | Catalyst | Catalysts 3 | Catalysts 4 | 283°C | 99.06% | 28 kPa |
| | Particle diameter D50S [mm] | 4.9 | 4.9 | | | |
| | Specific surface area A [m2/g] | 1.74 | 1.74 | | | |
| | D50S × A | 8.53 | 8.53 | | | |
| | Cumulative pore volume B [cm3/g] | 9.52E-04 | 9.52E-04 | | | |
| | Preliminarily calcined powder particle diameter D50T [μm] | 14.25 | 14.25 | | | |

[Examples 2 to 7 and Comparative Example 2]

Catalysts 5 to 11 were produced in order to study the catalytic activity applicable to the present invention.

<Production of Catalysts 5>

Spherical catalysts were produced in the same production method as that for the catalysts 1 except that the preliminarily calcined powder (E) was pulverized with a vibration mill such that the median diameter was 11.09 $\mu$m, the carrying and molding was carried out such that the median diameter D50S of the catalyst was 5.2 mm and the amount carried was 33 mass%, and the main calcination was carried out such that the temperature after 12 hours was 390°C. The specific surface area A was 1.63 m$^2$/g, the D50S × A was 8.48, and the D50S/A was 3.19.

<Production of Catalysts 6>

Spherical catalysts were produced in the same production method as that for the catalysts 1 except that the preliminarily calcined powder (E) was pulverized with a vibration mill such that the median diameter was 2.47 μm, the carrying and molding was carried out such that the median diameter D50S of the catalyst was 5.2 mm and the amount carried was 33 mass%, and the main calcination was carried out such that the temperature after 12 hours was 390°C. The specific surface area A was 1.64 m$^2$/g, the D50S × A was 8.53, and the D50S/A was 3.17.

<Production of Catalysts 7>

Spherical catalysts were produced in the same production method as that for the catalysts 1 except that the preliminarily calcined powder (E) was pulverized with a vibration mill such that the median diameter was 2.47 μm, the carrying and molding was carried out such that the median diameter D50S of the catalyst was 5.2 mm and the amount carried was 33 mass%, and the main calcination was carried out such that the temperature after 12 hours was 380°C. The specific surface area A was 1.75 m$^2$/g, the D50S × A was 9.10, and the D50S/A was 2.98.

<Production of Catalysts 8>

Spherical catalysts were produced in the same production method as that for the catalysts 1 except that the preliminarily calcined powder (E) was pulverized with a vibration mill such that the median diameter was 2.47 μm, the carrying and molding was carried out such that the median diameter D50S of the catalyst was 5.2 mm and the amount carried was 33 mass%, and the main calcination was carried out such that the temperature after 12 hours was 370°C. The specific surface area A was 1.73 m$^2$/g, the D50S × A was 9.01, and the D50S/Awas 3.01.

<Production of Catalysts 9>

Spherical catalysts were produced in the same production method as that for the catalysts 1 except that the preliminarily calcined powder (E) was pulverized with a vibration mill such that the median diameter was 2.47 μm, the carrying and molding was carried out such that the median diameter D50S of the catalyst was 5.2 mm and the amount carried was 33 mass%, and the main calcination was carried out such that the temperature after 12 hours was 360°C. The specific surface area A was 1.64 m$^2$/g, the D50S × A was 8.53, and the D50S/A was 3.17.

<Production of Catalysts 10>

Spherical catalysts were produced in the same production method as that for the catalysts 1 except that the preliminarily calcined powder (E) was pulverized with a vibration mill such that the median diameter was 5.38 μm, the carrying and molding was carried out such that the median diameter D50S of the catalyst was 5.2 mm and the amount carried was 33 mass%, and the main calcination was carried out such that the temperature after 12 hours was 355°C. The specific surface area A was 2.09 m$^2$/g, the D50S × A was 10.87, and the D50S/A was 2.49.

<Production of Catalysts 11>

Spherical catalysts were produced in the same production method as that for the catalysts 1 except that the preliminarily calcined powder (E) was pulverized with a vibration mill such that the median diameter was 37.10 μm, the carrying and molding was carried out such that the median diameter D50S of the catalyst was 5.2 mm, and the main calcination was carried out such that the temperature after 12 hours was 390°C. The specific surface area A was 1.22 m$^2$/g, the D50S × A was 6.34, and the D50S/A was 4.26.

<Acrylic Acid Production Evaluation>

Acrylic acid was produced by the following method using the catalysts 5 to 10 (Examples 2 to 7) and the catalyst 11 (Comparative Example 2).
An acrolein oxidation reaction (second stage) was carried out after a propylene oxidation reaction (first stage), and the acrolein conversion rate was determined. In both the first stage and the second stage, a stainless steel reaction tube having an inner diameter of 28.4 mm was filled with 68 mL of catalysts. In the first stage, a mixed gas containing 8 vol% of propylene, 67 vol% of air, and 25 vol% of water vapor was introduced for a contact time of 4 seconds to carry out the propylene oxidation reaction to produce acrolein. The gas produced in the reaction in the first stage was introduced into the second stage, the salt bath temperature in the second stage was set at 250°C, and the acrolein conversion rate was determined.

Table 2 shows evaluation results regarding the acrolein conversion rate (catalytic activity).

[Table 2]

| | Catalyst | Particle diameter D50S [mm] | Specific surface area A [m2/g] | D50S × A | Preliminarily calcined powder particle diameter D50T [μm] | Salt bath temperature [°C] | Acrolein conversion rate |
|---|---|---|---|---|---|---|---|
| Comparative Example 2 | Catalysts 11 | 5.2 | 1.22 | 6.34 | 37.10 | 250 | 99.46% |
| Example 2 | Catalysts 5 | 5.2 | 1.63 | 8.48 | 11.09 | 250 | 99.59% |
| Example 3 | Catalysts 6 | 5.2 | 1.64 | 8.53 | 2.47 | 250 | 99.63% |
| Example 4 | Catalysts 7 | 5.2 | 1.75 | 9.10 | 2.47 | 250 | 99.82% |
| Example 5 | Catalysts 8 | 5.2 | 1.73 | 9.00 | 2.47 | 250 | 99.88% |
| Example 6 | Catalysts 9 | 5.2 | 1.64 | 8.53 | 2.47 | 250 | 99.84% |
| Example 7 | Catalysts 10 | 5.2 | 2.09 | 10.87 | 5.38 | 250 | 99.77% |

[Reference Examples 1 and 2]

<Production of Catalyst 12>

Spherical catalysts were produced in the same production method as that for the catalysts 1 except that the glass fiber was not added in the carrying and molding stage for the preliminarily calcined powder (E).

<Abrasion Degree Evaluation>

The catalysts 1 and the catalysts 12 each in an amount of 50 g were charged into a cylindrical rotating machine having a radius of 14 cm, equipped with one baffle plate inside, followed by rotation at 23 rpm for 10 minutes, thereafter, the peeled-off powder was removed with a sieve, and the remaining amount was measured. The proportion of the peeled-off powder was calculated using the following equation (5). This value was expressed as the abrasion degree, and results were shown in Table 3.
[Equation (5)] Abrasion degree (mass%) = 100 × [(mass of sample - mass of sample remaining on sieve)/mass of sample]     (5)

[Table 3]

| | Catalyst | Abrasion degree [%] |
|---|---|---|
| Reference Example 1 | Catalyst 1 | 0.4 |
| Reference Example 2 | Catalyst 12 | 26.2 |

As seen from the results shown in Table 1, the use of the catalyst according to the present invention for the upper layer and the lower layer, allowed for achieving a reduction in differential pressure, and further allowed for maintaining the acrolein conversion rate at a high level. That is, this showed that a highly active catalyst can be used stably for a long period of time. In addition, it could be confirmed from Table 2 that the catalysts according to the present invention used in Examples 2 to 7 exhibited high catalytic activity. Further, it could be confirmed from Table 3 that addition of the glass fiber significantly improved the abrasion degree.

The present application is based on a Japanese Patent Application No. 2021-168460 filed on October 14, 2021, contents of which are incorporated herein by reference.

INDUSTRIAL APPLICABILITY

The present invention allows for reducing the differential pressure and maintaining the catalytic activity at a high level, when producing acrylic acid by subjecting acrolein as a raw material to a catalytic gas-phase oxidation reaction. Therefore, this allows a plant for producing acrylic acid to be stably operated for a long period of time, which is very useful.

**Claims**

1. Catalysts for producing an unsaturated carboxylic acid, comprising a catalytically active component represented by the following formula (1), wherein

   the catalysts have a median diameter D50S (mm) 5.0 mm or more and 8.0 mm or less, and
   a relationship between a specific surface area A ($m^2$/g) of the catalysts and the median diameter D50S (mm) of the catalysts is expressed by the following expression (2):

   $$(Mo)_{12}(V)_a(W)_b(Cu)_c(Sb)_d(X)_e(Y)_f(Z)_g(O)_h \qquad (1)$$

   $$8.0 \leq D50S \times A \leq 20.0 \qquad (2)$$

   where, Mo, V, W, Cu, Sb, and O represent molybdenum, vanadium, tungsten, copper, antimony, and oxygen respectively; X represents at least one element selected from the group consisting of an alkali metal and thallium; Y represents at least one element selected from the group consisting of magnesium, calcium, strontium, barium, and zinc; and Z represents at least one element selected from the group consisting of niobium, cerium, tin, chromium, manganese, iron, cobalt, samarium, germanium, titanium, and arsenic; a, b, c, d, e, f, g, and h indicate an atomic ratio of respective element to a molybdenum atom being 12, and $0 < a \leq 10$, $0 \leq b \leq 10$, $0 < c \leq 6$, $0 \leq d \leq 10$, $0 \leq e \leq 0.5$, $0 \leq f \leq 1$, and $0 \leq g < 6$; and h is the number of oxygen atoms required to satisfy a valence of each component described above.

2. The catalysts for producing an unsaturated carboxylic acid according to claim 1, wherein the relationship between the specific surface area A ($m^2$/g) and the median diameter D50S (mm) is expressed by the following expression (3):

   $$8.6 \leq D50S \times A \leq 20.0 \qquad (3).$$

3. The catalysts for producing an unsaturated carboxylic acid according to claim 1 or 2, wherein a cumulative pore volume B ($cm^3$/g) measured by a nitrogen adsorption method is $9.70 \times 10^{-4}$ $cm^3$/g or more and $2.00 \times 10^{-3}$ $cm^3$/g or less.

4. The catalysts for producing an unsaturated carboxylic acid according to any one of claims 1 to 3, wherein $1 \leq a \leq 5$, $0.5 \leq b \leq 3$, $0.5 \leq c \leq 3$, and $0 < d \leq 2$ are satisfied in the formula (1).

5. The catalysts for producing an unsaturated carboxylic acid according to any one of claims 1 to 4, wherein catalyst precursors containing the catalytically active component are carried on inert carriers.

6. The catalysts for producing an unsaturated carboxylic acid according to claim 5, wherein the inert carrier is silica, alumina, or a combination thereof.

7. The catalysts for producing an unsaturated carboxylic acid according to any one of claims 1 to 6, further comprising: an inorganic fiber.

8. A method for producing the catalysts for producing an unsaturated carboxylic acid according to claim 5 or 6, comprising:

carrying the catalyst precursors on the inert carriers, wherein
the catalyst precursors have a median diameter D50T of 1.0 $\mu$m or more and 14.0 $\mu$m or less.

9. The method for producing the catalysts for producing an unsaturated carboxylic acid according to claim 8, further comprising:
carrying out calcination at a temperature of 350°C or higher and 380°C or lower, after carrying the catalyst precursors on the inert carriers.

10. A method for producing an unsaturated carboxylic acid using the catalysts for producing an unsaturated carboxylic acid according to any one of claims 1 to 7.

11. A method for producing an unsaturated carboxylic acid comprising, using a reaction tube filled with two or more of the catalysts for producing an unsaturated carboxylic acid according to any one of claims 1 to 7 in multiple layers.

12. The method for producing an unsaturated carboxylic acid according to claim 10 or 11, wherein a difference between a reactor inlet pressure and a reactor outlet pressure during a reaction is 27 kPa or less.

## INTERNATIONAL SEARCH REPORT

| International application No. |
| --- |
| **PCT/JP2022/038037** |

### A. CLASSIFICATION OF SUBJECT MATTER

**B01J 23/888**(2006.01)i; **B01J 35/10**(2006.01)i; **B01J 37/08**(2006.01)i; **C07B 61/00**(2006.01)i; **C07C 45/35**(2006.01)i; **C07C 47/22**(2006.01)i; **C07C 51/235**(2006.01)i; **C07C 57/055**(2006.01)i
FI:   B01J23/888 Z; B01J35/10 301G; B01J37/08; C07B61/00 300; C07C45/35; C07C47/22 C; C07C51/235; C07C57/055 A

According to International Patent Classification (IPC) or to both national classification and IPC

### B. FIELDS SEARCHED

Minimum documentation searched (classification system followed by classification symbols)
B01J23/888; B01J35/10; B01J37/08; C07B61/00; C07C45/35; C07C47/22; C07C51/235; C07C57/055

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 64-063543 A (NIPPON SHOKUBAI KAGAKU KOGYO CO LTD) 09 March 1989 (1989-03-09)<br>claims 1-11, p. 6, upper right column, line 15 to p. 7, lower left column, line 7, p. 8, lower left column, line 1 to p. 13, upper right column, p. 14 | 1-12 |
| A | JP 2017-124384 A (MITSUBISHI CHEM CORP) 20 July 2017 (2017-07-20)<br>claims 1-7, paragraphs [0046]-[0047] | 1-12 |
| A | WO 2015/053269 A1 (NIPPON KAYAKU KABUSHIKI KAISHA) 16 April 2015 (2015-04-16)<br>entire text | 1-12 |
| A | JP 2020-157294 A (NIPPON KAYAKU KABUSHIKI KAISHA) 01 October 2020 (2020-10-01)<br>entire text | 1-12 |
| A | JP 2020-179312 A (NIPPON KAYAKU KABUSHIKI KAISHA) 05 November 2020 (2020-11-05)<br>entire text | 1-12 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **18 November 2022** | **06 December 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/038037**

| Patent document cited in search report | Publication date (day/month/year) | Patent family member(s) | Publication date (day/month/year) |
|---|---|---|---|
| JP 64-063543 A | 09 March 1989 | US 5218146 A<br>claims 1-9, column 7, line 29<br>to column 9, line 8, column 10,<br>line 51 to column 17, line 8<br>EP 293224 A1<br>KR 10-1988-0013870 A<br>CN 1030228 A | |
| JP 2017-124384 A | 20 July 2017 | (Family: none) | |
| WO 2015/053269 A1 | 16 April 2015 | US 2016/0244393 A1<br>entire text<br>EP 3056482 A1<br>CN 105612141 A<br>KR 10-2016-0068763 A | |
| JP 2020-157294 A | 01 October 2020 | (Family: none) | |
| JP 2020-179312 A | 05 November 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H08299797 A
- JP 2003251184 A
- JP 2015120133 A
- JP 2001079408 A
- WO 2012073584 A
- JP 2021168460 A